(19) Europäisches Patentamt
European Patent Office
Office européen des brevets

(11) EP 0 562 480 B1

(12) EUROPEAN PATENT SPECIFICATION

(45) Date of publication and mention of the grant of the patent:
**02.01.1997 Bulletin 1997/01**

(21) Application number: **93104528.0**

(22) Date of filing: **19.03.1993**

(51) Int Cl.[6]: **C07D 249/10**, C07D 249/14, C07D 409/06, A01N 43/653, A01N 43/84
// C07D263/42, C07D263/48, C07D413/06

(54) **1-phenyl-5-substituted-1H-1, 2,4-triazole-3-carboxamide derivates, process for preparation of the same, and fungicidal composition comprising the same**

1-Phenyl-5-substituierte 1H-1,2,4-Triazol-3-Carboxamidderivate, Verfahren zu ihrer Herstellung und diese enthaltende fungizide Zusammensetzungen

Dérivés de 1-phényl-5-substituées 1H-1,2,4-triazole-3-carboxamide, procédé pour leur préparation et composition fongicide les contenant

(84) Designated Contracting States:
**DE FR GB**

(30) Priority: **27.03.1992 JP 102147/92**

(43) Date of publication of application:
**29.09.1993 Bulletin 1993/39**

(73) Proprietor: **KUREHA CHEMICAL INDUSTRY CO., LTD.**
**Chuo-ku Tokyo 103 (JP)**

(72) Inventors:
- **Watanabe, Takeo**
  **Iwaki-shi, Fukushima (JP)**
- **Saishoji, Toshihide**
  **Iwaki-shi, Fukushima (JP)**
- **Shida, Takafumi**
  **Iwaki-shi, Fukushima (JP)**

(74) Representative: **Boeters, Hans Dietrich, Dr. et al**
**Patentanwälte Boeters & Bauer,**
**Bereiteranger 15**
**81541 München (DE)**

(56) References cited:
**EP-A- 0 174 562**
**EP-A- 0 189 300**
**EP-A- 0 220 956**
**EP-A- 0 282 303**
**EP-A- 0 282 669**

- **PATENT ABSTRACTS OF JAPAN, vol. 16, no. 461 (C-988), 25 September 1992**

Remarks:
The file contains technical information submitted after the application was filed and not included in this specification

EP 0 562 480 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

### BACKGROUND OF THE INVENTION

#### Field of the Invention

The present invention relates to a derivative of 1-phenyl-5-substituted-1H-1,2,4-triazole-3-carboxamide, to a process for manufacturing the same, and to a fungicidal composition comprising the same.

#### Description of Background Art

EP-A-0 174 562 discloses 1-phenyl-5-substituted 1H-1,2,4-triazole-3-carboxamide derivatives carrying a phenyl substituent at position 1 wherein the phenyl substituent may be substituted by a haloalkyl group.

JP-A-04 164 073 concerns 1-phenalkyl-3-phenyl-1H-1,2,4-triazole-5-carboxamide derivatives.

EP-A-0 189 300 describes 4,5-dihydro-1-phenyl-5-substituted 1H-1,2,4-triazole-3-carboxamide derivatives and EP-A-0 220 956, EP-A-0 282 303 and EP-A-0 282 669 disclose 1,5-diphenyl-1H-1,2,4-triazole-3-carboxamide derivatives. There has, however, still been a demand for additional and effective fungicidal compositions.

While said derivatives of 1,5-diphenyl-1H-1,2,4-triazole-3-carboxamide with a substituted or unsubstituted alkoxymethyl group bonded to the 3 position of 1-phenyl group of the triazole ring described in the above prior arts are useful as a herbicidal composition, the present inventors have discovered that a novel compound of which hydrogen atom of the methylene group is further substituted, as shown in the following formula (I), is useful as a fungicidal agent.

### SUMMARY OF THE INVENTION

Accordingly, an object of the present invention is to provide a novel derivative of 1-phenyl-5-substituted-1H-1,2,4-triazole-3-carboxamide represented by the following formula (I),

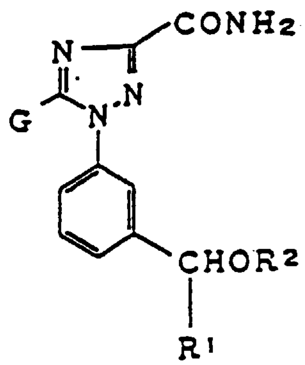

(I)

wherein $R^1$ is a $C_1$-$C_6$ alkyl group, $R^2$ is a $C_1$-$C_8$ alkyl group, and G represents a $C_1$-$C_8$ alkyl group, a $C_1$-$C_4$ fluoroalkyl group, a $C_3$-$C_7$ cycloalkyl group, a phenyl $C_1$-$C_4$ alkyl group, a phenyl $C_2$-$C_4$ alkenyl group, a morpholino group, or a thenyl group.

Another object of the present invention is to provide a process for manufacturing said novel carboxamide derivative.

Still another object of the present invention is to provide a fungicidal composition, especially a fungicidal composition used for agriculture and horticulture, comprising said novel carboxamide derivative.

Specifically, the present invention comprises the following features as an active ingredient.

A first invention relates to a derivative of 1-phenyl-5-substituted-1H-1,2,4-triazole-3-carboxamide (I),

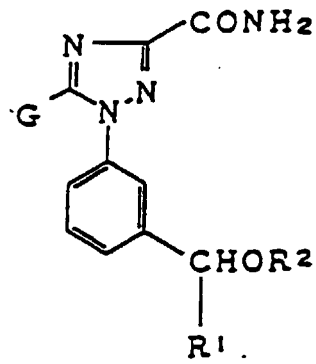

(I)

wherein $R^1$ is a $C_1$-$C_6$ alkyl group, $R^2$ is a $C_1$-$C_8$ alkyl group, and G represents a $C_1$-$C_8$ alkyl group, a $C_1$-$C_4$ fluoroalkyl group, a $C_3$-$C_7$ cycloalkyl group, a phenyl $C_1$-$C_4$ alkyl group, a phenyl $C_2$-$C_4$ alkenyl group, a morpholino group, or a thenyl group.

A second invention relates to a process for manufacturing said derivative of 1-phenyl-5-substituted-1H-1,2,4-triazole-3-carboxamide (I), which comprises reacting oxazoledione hydrazone derivative (VI) and ammonia to produce glycinamide derivative (VII) and subjecting the glycinamide derivative (VII) to a cyclocondensation reaction according to the following reaction formula (I),

**Reaction (I)**

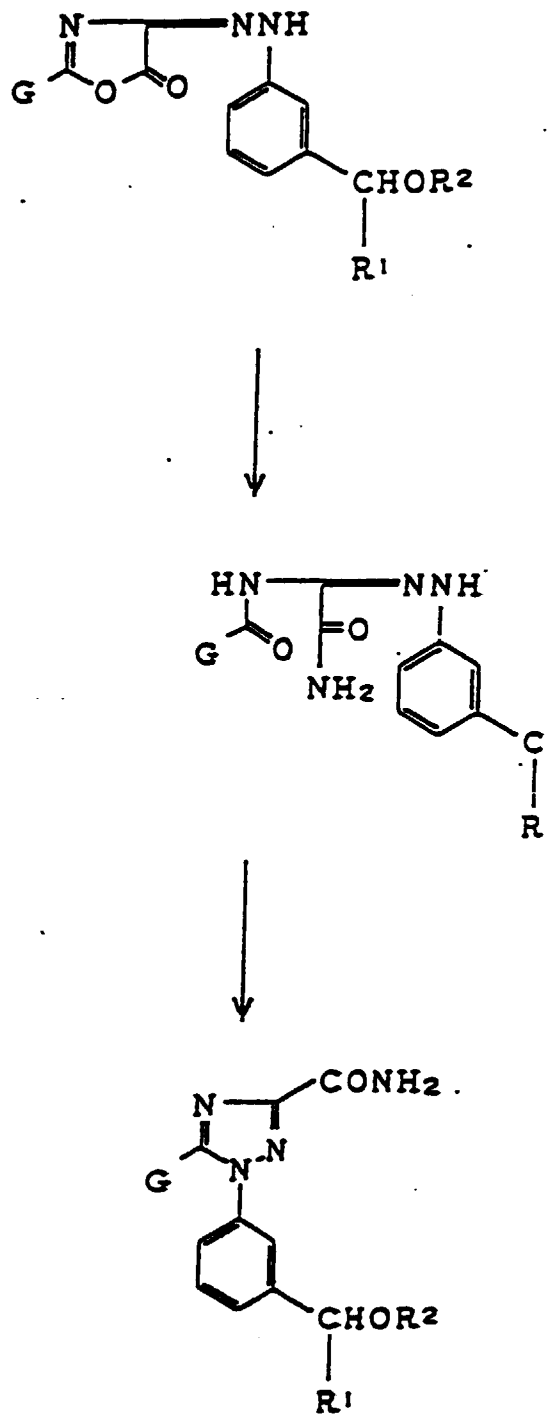

(VI)

(VII)

(I)

wherein R[1], R[2], and G has the same meanings as defined above.

A third invention relates to a process far manufacturing said derivative of 1-phenyl-5-substituted-1H-1,2,4-triazole-3-carboxamide (I), which comprises reacting oxamide derivative (X) and aldehyde derivative (XI) to produce dihydrotriazole carboxamide (XII) and oxidizing the dihydrotriazole carboxamide (XII) according to the following reaction formula (II),

**Reaction (II)**

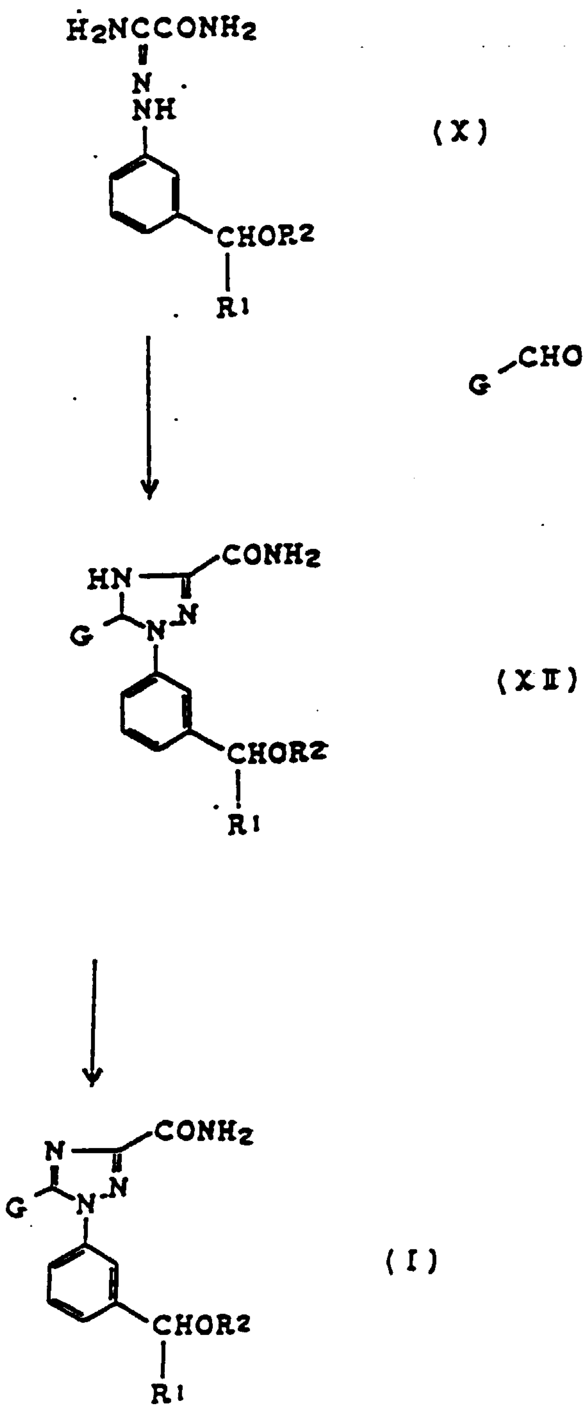

(X)

(XI)

(XII)

(I)

wherein $R^1$, $R^2$ and G has the same meanings as defined above.

A fourth invention relates to a fungicidal composition comprising said derivative of 1-phenyl-5-subsituted-1H-1,2,4-triazole-3-carboxamide (I) as an active ingredient.

Other objects, features and advantages of the invention will hereinafter becomes more readily apparent from the following description.

DETAILED DESCRIPTION OF THE INVENTION AND PREFERRED EMBODIMENTS

Given as specific examples of derivatives of 1-phenyl-5-substituted-1H-1,2,4-triazole-3-carboxamide (I) are compounds in the following Table 1.

TABLE 1

| | Substituents | Physicochemical characteristics |
|---|---|---|
| Compound No. | $R^1$ | Appearance |
| | $R^2$ | Melting point (°C) |
| | G | IR ($cm^{-1}$) |
| 1 | $(CH_3)_2CH$<br>$CH_3CH_2CH_2CH_2$<br>$CH_3CH_2$ | Light brown liquid<br><br>3450 3260 1685 |
| 2 | $(CH_3)_2CH$<br>$CH_3CH_2CH_2CH_2$<br>$(CH_3)_2CHCH_2$ | Colorless crystal<br>47-49<br>3455 3280 1685 |
| 3 | $(CH_3)_2CH$<br>$CH_3CH_2CH_2CH_2$<br>$CH_3CH_2CH_2CH_2CH_2$ | Light brown liquid<br><br>3450 3300 1680 |
| 4 | $(CH_3)_2CH$<br>$CH_3CH_2CH_2CH_2$<br>$CF_3CF_2CF_2$ | Light brown liquid<br><br>3460 3275 1675 |
| 5 | $(CH_3)_2CH$<br>$CH_3CH_2CH_2CH_2$<br>Cyclohexyl | Light brown liquid<br><br>3450 3300 1680 |
| 6 | $(CH_3)_2CH$<br>$CH_3CH_2CH_2CH_2$<br>$PhCH_2$ | Light brown liquid<br><br>3450 3275 1670 |
| 7 | $(CH_3)_2CH$<br>$CH_3CH_2CH_2CH_2$<br>$CH_3(Ph)CH$ | Colorless crystal<br>94-96<br>3360 3200 1680 |
| 8 | $(CH_3)_2CH$<br>$CH_3CH_2CH_2CH_2$<br>$PhCH_2CH_2$ | Light brown liquid<br><br>3455 3300 1680 |
| 9 | $(CH_3)_2CH$<br>$CH_3CH_2CH_2CH_2$<br>PhCH=CH | Light brown liquid<br><br>3455 3290 1680 |
| 10 | $(CH_3)_2CH$<br>$CH_3CH_2CH_2CH_2$<br>Morpholino | Light brown liquid<br><br>3400 3300 1675 |
| 11 | $(CH_3)_2CH$<br>$CH_3CH_2CH_2CH_2$<br>Thenyl | Gray crystal<br>82-84<br>3350 3150 1655 |

Since compound (I) has a triazole ring, it may be used in forms such as inorganic acid salts, organic acid salts or metal complexes. The present invention includes such salts and complexes.

In addition, there are optical isomers for compound (I) due to the substituent at 3 position of 1-phenyl group. All of such optical isomers as well as mixtures of isomers at any ratio are also included in the present invention.

The following processes (A) or (B) can be employed for manufacturing 1-phenyl-5-substituted-1H-1,2,4-triazole-3-carboxamide.

<Process (A)>

According to the above reaction formula (I), oxazoledione hydrazone derivative (VI) and ammonia are reacted to produce glycineamide derivative (VII). The glycineamide derivative (VII) is converted into compound (I) by a cyclocondensation reaction in an organic solvent in the presence of an acid catalyst.

<Process (B)>

According to the above reaction formula (II), oxamide derivative (X) and aldehyde derivative (XI) are reacted in an organic solvent to produce dihydrotriazole carboxamide (XII). The dihydrotriazole carboxamide (XII) is oxidized into compound (I).

Processes for producing the oxazoledione hydrazone derivative of formula (VI) used in Process (A) are discussed in detail.

<Diazotization and diazo coupling reaction>

According to the reaction (III) shown below, aniline derivative (II) is converted into diazonium salt derivative (III) by adding dropwise an aqueous solution of nitrite such as sodium nitrite to a mixture of the aniline derivative (II), an inorganic acid, e.g., hydrochloric acid or sulfuric acid, and an organic acid, e.g., acetic acid, or propionic acid, while cooling and with stirring. The diazonium salt derivative (III) thus obtained is reacted with a glycine derivative (IV), acetic anhydride, and a basic compound to produce oxazoledione hydrazone derivative (VI). Given as basic compounds used here are alkali metal salts of weak acid (e.g., sodium acetate), alkaline earth metal salts of weak acid (e.g., calcium acetate), alkaline earth metal oxides (e.g., calcium oxide, magnesium oxide), and tertiary amines (e.g., pyridine derivatives, triethylamine, tripropylamine). Alternatively, glycine derivative (IV) may be used for the diazo coupling reaction after converting it into oxazolone derivative (V) by heating together with acetic anhydride.

**Reaction (III)**

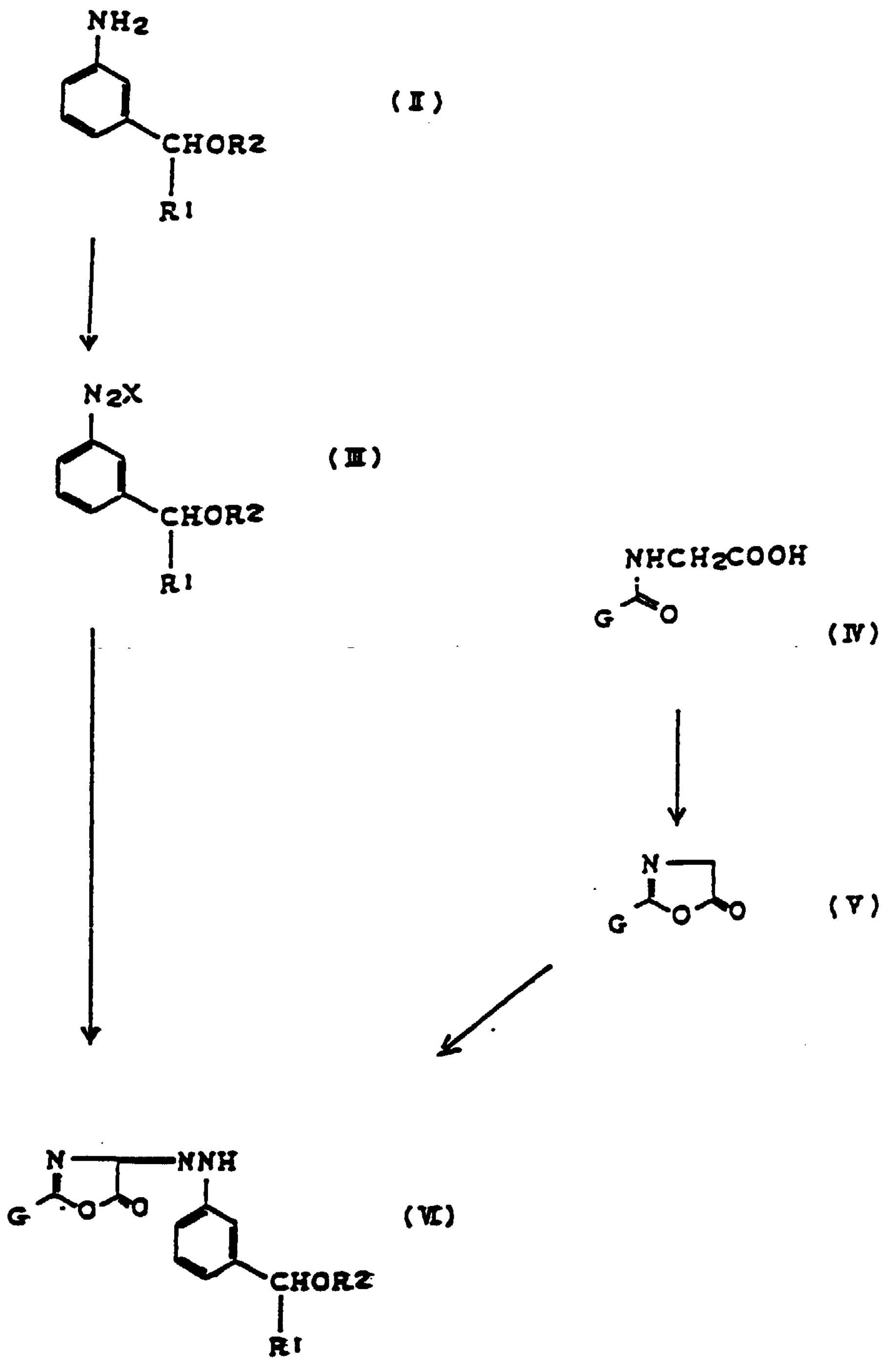

wherein $R^1$, $R^2$, and G are the same as defined above, and X denotes an anion such as chlorine ion, sulfate ion, or boron tetrafluoride ion.

<Acylation reaction, nitration reaction, and reduction of carbonyl group>

Aniline derivative (II) can be obtained via nitrobenzyl alcohol derivative (XVII) as shown in the reaction (IV) below.

Benzene derivative (XIII) is acylated with acyl halide (XIV) or carboxylic acid anhydride in the presence of Lewis acid, e.g., $AlCl_3$, $AlBr_3$, $ZnCl_2$, or $BF_3$, preferably $AlCl_3$, to produce ketone derivative (XV). In this reaction, an excessive amount of the reaction substrate or an organic solvent, e.g., nitrobenzene or carbon disulfide, can be used as a reaction solvent. The ketone derivative (XV) is converted into nitrophenyl ketone derivative (XVI) by nitration using nitric acid in a mixture of nitric acid and sulfuric acid or in acetic acid solvent. Nitrobenzyl alcohol derivative (XVII) can be produced by reducing the carbonyl group of the nitrophenyl ketone derivative (XVI) thus obtained. The use of sodium borohydride as a reducing agent and an alcohol, e.g., ethyl alcohol, is preferred for the reducing reaction.

**Reaction (IV)**

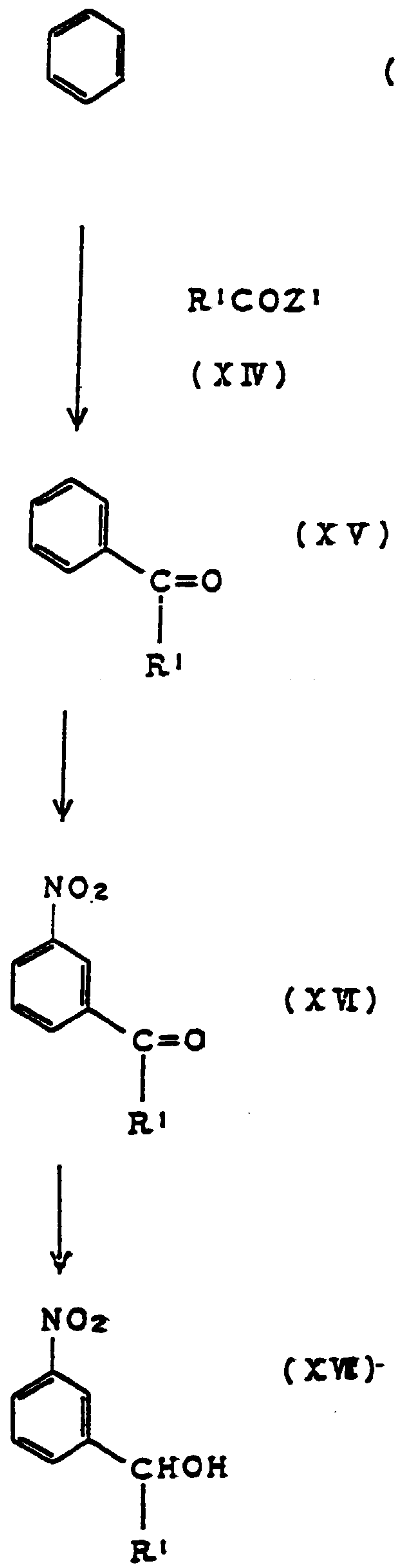


(XIII)

(XV)

(XVI)

(XVII)

wherein $R^1$, $R^2$, and G are the same as defined above, and $Z^1$ represents a halogen atom or a group $R^1COO$, preferably

Cl or Br.

<Etherification reaction and nitro group reducing reaction>

According to the reaction (V) shown below, nitrobenzyl alcohol derivative (XVII) is etherified with alkylating agent, such as alkyl halide (XVIII) or alkyl sulfonate, to produce nitrobenzyl ether derivative (XIX). An acid-binding agent used in this reaction is a basic compound such as, for example, hydroxide or carbonate of alkali metal or a hydroxide, oxide or carbonate of alkaline earth metal, preferably potassium hydroxide, sodium hydride or potassium carbonate. The reaction is carried out preferably in the presence of a solvent, such as an aprotic polar solvent, e.g., dimethylformamide, dimethylacetamide, N-methylpyrrolidone, dimethylsulfoxide, tetrahydrofuran, sulfolane, or acetonitrile. The reaction temperature is usually -10 to 100°C, and the reaction time is 0.5 to 25 hours, and preferably 1 to 10 hours. The nitro group in the nitrobenzyl ether derivative (XIX) thus obtained is reduced to produce aniline derivative (II). A Bechamp reduction agent, e.g., hydrochloric acid and metallic iron; stannous chloride; catalytic hydrogenation agent, e.g., hydrogen molecule catalyzed by platinum/activated charcoal or palladium/activated charcoal ; or hydrazine hydrate catalyzed by activated charcoal and ferric chloride; can be used in the reduction. The solvent used differs depending on the reduction method employed. An organic acid, e.g., acetic acid or propionic acid, can be used for the Bechamp reduction; an alcohol, e.g., ethyl alcohol or isopropyl alcohol or an aromatic hydrocarbon, e.g., benzene or toluene, can be used for the catalytic reduction. The reaction temperature is usually -10 to 100°C, and the reaction time is 0.5 to 24 hours, and preferably 1 to 10 hours.

Reaction (V)

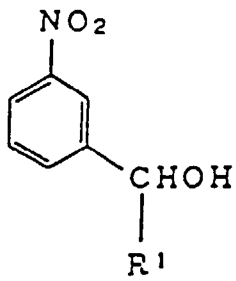

(XVII)

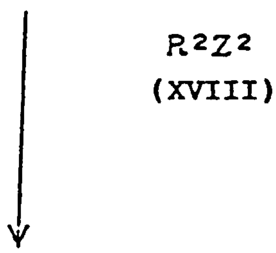

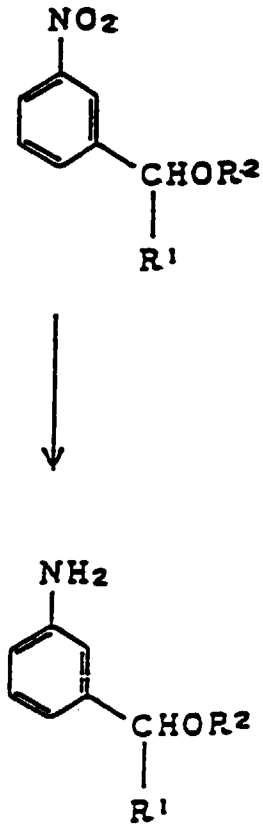

(XIX)

(II)

wherein $R^1$, $R^2$, and G are the same as defined above, and $Z^2$ represents a halogen atom or a group $QSO_2O$, wherein Q is a $C_1$-$C_4$ alkyl group, substituted or unsubstituted phenyl group, preferably methyl group, phenyl group, or p-methylphenyl group.

Process (B) is now discussed in detail.

<Diazotization and Japp-Klingemann reaction>

Oxamide derivative (X) can be prepared as follows. According to the reaction (VI) shown below, aniline derivative (II) is converted into diazonium salt derivative (III) by adding dropwise an aqueous solution of nitrite such as sodium nitrite into a mixture of the aniline derivative (II) and an inorganic acid (e.g., hydrochloric acid, sulfuric acid), and a lower alcohol (e.g., methyl alcohol), while cooling and stirring. The diazonium salt derivative (III) is then reacted with 2-chloroacetoacetate derivative (VIII) and a basic compound, preferably in a lower alcohol such as methanol , to obtain oxalyl chloride derivative (IX).

Given as basic compounds used here are alkali metal salts of weak acid (e.g., sodium acetate), alkaline earth metal salts of weak acid (e.g., calcium acetate), alkaline earth metal oxides (e.g., calcium oxide, magnesium oxide), tertiary amines (e.g., pyridine derivatives, triethylamine, tripropylamine).

The oxalyl chloride derivative and ammonia are reacted in a lower alcohol such as methanol at -20 to 50°C for 10 to 30 hours to obtain oxamide derivative (X).

Reaction (VI)

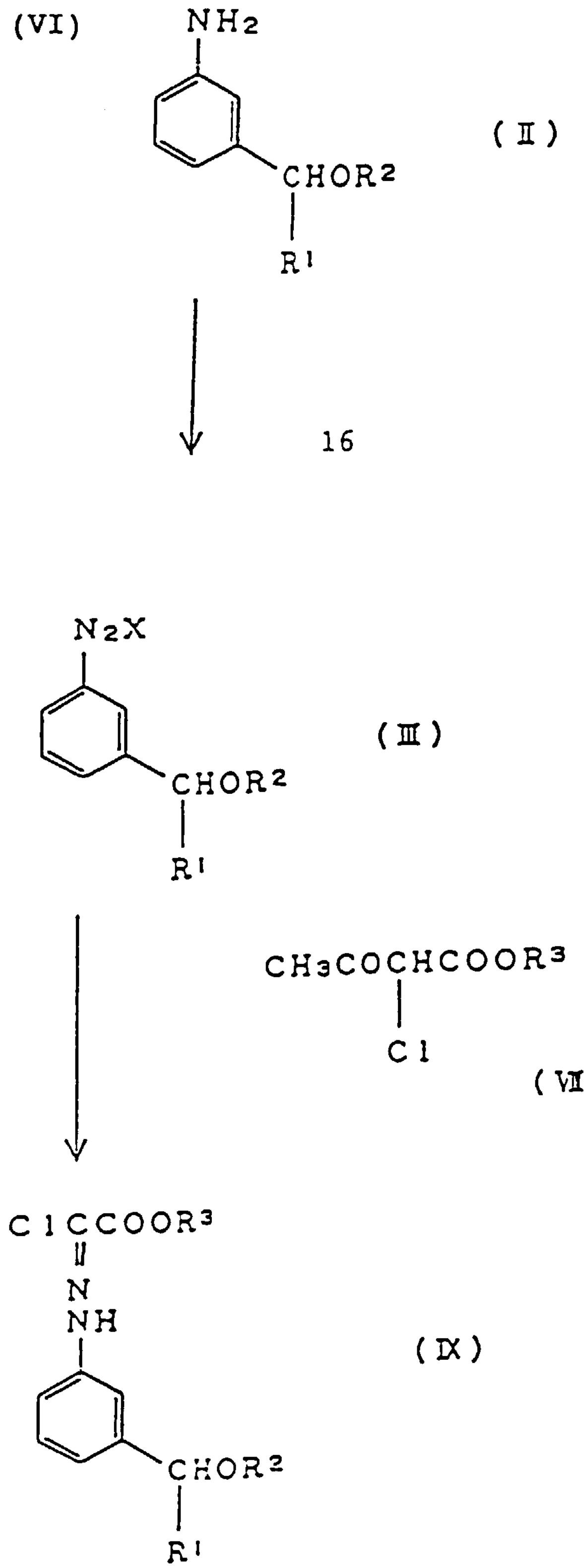


(II)

(III)

(VII)

(IX)

wherein $R^1$, $R^2$, and G are the same as defined above, and $R^3$ represents a $C_1$-$C_4$ alkyl group, and X denotes an anion such as chlorine ion, sulfate ion or boron tetrafluoride ion.

<Cyclocondensation reaction and auto-oxidation reaction>

As shown in the reaction formula (II), oxamide derivative (X) and benzaldehyde derivative (XI) are reacted in a lower fatty acid such as acetic acid, or propionic acid to obtain dihydrotriazole carboxamide (XII), followed by auto-oxidation of the dihydrotriazole carboxamide. A cyclocondensation reaction takes place along with and the auto-oxidation reaction at a temperature of 10 to 40°C and reaction time for 10 to 30 hours to produce a derivative of 1-phenyl-5-substituted-1H-1,2,4-triazole-3-carboxamide (I).

When the derivative of l-phenyl-5-substituted-1H-1,2,4-triazole-3-carboxamide (I) manufactured by Process (A) or Process (B) is used as a fungicidal agent, especially as a fungicidal composition used for agriculture and horticulture, the compound is usually made into a formulation, together with adjuvants, in the form of powder, wettable or water-dispersible powder, granule or emulsion concentrate, although the compound may be used as is.

Such a formulation may contain one or more compounds of the present,invention in an amount of 0.1 to 95%, preferably 0.5 to 90%, and more preferably 2 to 70% by weight.

Examples of carriers, diluents and surfactants which can be used as adjuvants in the composition of the present invention are as follows. Talc, kaolin, bentonite, diatomaceous earth, white carbon or clay, can be used as solid carriers; and water, xylene, toluene, chlorobenzene, cyclohexane, cyclohexanone, dimethylsulfoxide, dimethylformamide, or alcohols are used as diluents. Different types of surfactants may be used depending on the effects intended; e.g., polyoxyethylene alkylaryl ether, or polyoxyethylene sorbitan monolaurate, as an emulsifier; lignin sulfonate or dibutylnaphthalene sulfonate, as a dispersant; alkyl sulfonate, or alkylphenyl sulfonate, as a wetting agent.

Depending on the types such formulations are used as are or after having been diluted to a prescribed concentration with a diluent such as water.

When the formulation is used after dilution, its concentration is preferably in the range of 0.001 to 1.0%.

The compound of the present invention may be applied to fields, orchards or greenhouses, in an amount of 20 to 5,000 g, preferably 50 to 1,000 g, per 1 hector.

The concentrations and amounts of use may be varied depending on types of the formulation, the time at which it is applied, the manner and the site in which it is used, and the plant to which it is applied. Thus, the amount and concentration may be increased or decreased without regard to the above specified range.

In addition, the compound of the present invention can be used in combination with other active ingredients such as, for example, fungicides, bactericides, insecticides, acaricides or herbicides.

## EXAMPLES

### Synthetic Example 1

Synthesis of 1-[3-[(1-butoxy-2-methyl)propyl]phenyl]-5-cyclohexyl-1H-1,2,4-triazole-3-carboxamide: Compound 5, $R^1=(CH_3)_2CH$, $R^2=CH_3CH_2CH_2CH_2$, G=cyclohexyl in formula (I)

<Synthesis by Process (B)>

Diazotization Reaction

50 ml of methanol, 22.1 g (99.8 mmol) of 3-[(1-butoxy-2-methyl)propyl]aniline [$R^1=(CH_3)_2CH$, $R^2=CH_3CH_2CH_2CH_2$ in formula (II)], and 23.5 ml (264.8 mmol] of 35% hydrochloric acid were charged into a 200 ml conical flask and cooled on an ice water bath. To the mixture was added dropwise an aqueous solution of 7.3 g (105.8 mmol) of $NaNO_2$ dissolved in 10 ml of water to produce 3-[(1-butoxy-2-methyl)propyl]benzenediazonium chloride [$R^1$-$(CH_3)_2CH$, $R^2$-$CH_3CH_2CH_2CH_2$ in formula (III)]. Japp-Klingemann Reaction

15 g (99.6 mmol) of methyl chloroacetoacetate [$R^3=CH_3$ in formula (VIII)], 50 ml of methanol, and 20 g (243.8 mmol) of sodium acetate were measured and charged into a 500 ml conical flask, and cooled on an ice water bath. After the dropwise addition of the diazonium chloride solution prepared above, the mixture was reacted for 1 hour. The ice water bath was dismantled and the reaction was continued for a further 5 hours at room temperature. The precipitated product was collected by filtration, washed with water, and dried in air to obtain 32.3 g (94.8 mmol) of methyl chloro[[3-[(1-butoxy-2-methyl)propyl]phenyl]hydrazono]acetate [$R^1=(CH_3)_2CH$ $R^2=CH_3CH_2CH_2CH_2$, $R^3=CH_3$ in formula (IX)] at an yield of 95.2%.

Ammonolysis

17 g (49.9 mmol) of methyl chloro[[3-[(1-butoxy-2-methyl)propyl]phenyl]hydrazono]acetate was weighed and charged into a 300 ml eggplant type flask. After the addition of 100 ml of methanol containing 13% of ammonia, the

flask was capped and allowed to stand still at room temperature for 24 hours. The reaction liquid thus obtained was concentrated and shaken thoroughly with the addition of ethyl acetate, washed with water, and dried to quantitatively obtain oxamide [3-[(1-butoxy-2-methyl)propyl]phenyl]hydrazone [$R^1=(CH_3)_2CH$, $R^2=CH_3CH_2CH_2CH_2$, in formula (X)].

Cyclocondensation Reaction and Auto-oxidation Reaction

3.0 g (9.8 mmol) of oxamide [3-[(1-butoxy-2-methyl)propyl]phenyl]hydrazone was weighed and charged into a 50 ml conical type flask, followed by the addition of 30 ml of acetic acid to dissolve. To the solution was added 1.15 g (10.3 mmol) of cyclohexanecarboxaldehyde [G=cyclohexyl group in formula (XI)] and the mixture was allowed to stand at room temperature overnight.

The resultant reaction mixture was concentrated, thoroughly shaken with the addition of ethyl acetate, washed with water, dried, and concentrated to produce a crude product. This crude product was purified by column chromatography using a 10:1 (v/v) mixture of chloroform and acetone as a solvent to obtain 2.4 g of the title compound, 1-[3-[(1-butoxy-2-methyl)propyl]phenyl]-5-cyclohexyl-1H-1,2,4-triazole-3-carboxamide: Compound 5, $R^1=(CH_3)_2CH$, $R^2=CH_3CH_2CH_2CH_2$, G=cyclohexyl group in formula (I), at an yield of 61.0%.

The physicochemical characteristics of Compound 5 are shown in Table 1.

**Synthetic Example 2**

Synthesis of 1-[3-[(1-butoxy-2-methyl)propyl]phenyl]-5-(phenylmethyl)-1H-1,2,4-triazole-3-carboxamide: Compound 6, $R^1=(CH_3)_2CH$, $R^2=CH_3CH_2CH_2CH_2$, $G=PhCH_2$ in formula (I) Cyclocondensation Reaction and Auto-oxidation Reaction

3.0 g (9.8 mmol) of oxamide [3-[(1-butoxy-2-methyl)propyl]phenyl]hydrazone [$R^1=(CH_3)_2CH$, $R^2=CH_3CH_2CH_2CH_2$ in formula (X)] was weighed and charged into a 50 ml conical flask, followed by the addition of 30 ml of acetic acid to dissolve. To the solution was added 1.23 g (10.3 mmol) of phenylacetaldehyde [$G=PhCH_2$ in formula (XI)] and the mixture was allowed to stand at room temperature overnight. .

The resultant reaction mixture was concentrated, thoroughly shaken with the addition of ethyl acetate, washed with water, dried, and concentrated to produce a crude product. This crude product was purified by column chromatography using a 10:1 (v/v) mixture of chloroform and acetone as a solvent to obtain 1.8 g of the title compound, 1-[3-[(1-butoxy-2-methyl)propyl]phenyl]-5-(phenylmethyl)-1H-1,2,4-triazole-3-carboxamide: Compound 6, $R^1=(CH_3)_2CH$, $R^2=CH_3CH_2CH_2CH_2$, $G=PhCH_2$ in formula (I), at an yield of 46.0%.

The physicochemical characteristics of Compound 6 are shown in Table 1.

**Synthetic Example 3** (Synthesis of an intermediate compound)

Synthesis of 3-[(1-butoxy-2-methyl)propyl]aniline [$R^1=(CH_3)_2CH$, $R^2=CH_3CH_2CH_2CH_2$ in formula (II)]

a) Synthesis of 2-methyl-1-phenylpropanone [$R^1=(CH_3)_2CH$ in formula (XV)]

250 ml of dichloromethane was charged into a 1 l reaction flask, followed by the addition of 67 g (502.5 mmol) of $AlCl_3$. After cooling the mixture on an ice water bath to 5°C, 53.3 g (500.2 mmol) of isobutyryl chloride [$R^1=(CH_3)_2CH$, Z=Cl in formula (XIV)] was added dropwise.

Then, 39.06 g (500 mmol) of benzene was added dropwise, while maintaining the temperature at 5°C and the mixture was reacted for 2 hours at 5°C and 1 hour at 10°C, followed by refluxing for 1 hour. The resulting reaction mixture was poured into ice water, washed with water, dried, and concentrated to obtain a crude product. This crude product was distilled under vacuum to obtain 64.7 g (436.5 mmol) of 2-methyl-1-phenylpropanone [$R^1-(CH_3)_2CH$ in formula (XV)] as colorless oil at an yield of 87.3%.

b) Synthesis of 2-methyl-1-(3-nitrophenyl)propanone [$R^1=(CH_3)_2CH$ in formula (XVI)]

150 ml of 95% sulfuric acid was charge into a 500 ml reaction flask and cooled to -20°C on a dry ice-acetone bath. 15 ml of a concentrated nitric acid (61%, d=1.38) was added dropwise in portions and the mixture was cooled to -20°C. To this was added dropwise in portions 29-6 g (199.7 mmol) of 2-methyl-1-phenylpropanone so as not to raise the temperature above -5°C. The mixture was reacted for 1 hour at -5 to -10°C. After the reaction, the resulting reaction mixture was poured into ice water, extracted with ethyl acetate, washed with water, and dried to concentrate, thus obtaining 38.0 g (196.7 mmol) of a light brown oily product of 2-methyl-1-(3-nitrophenyl)propanone at an yield of 98.5%.

c) Synthesis of α-(1-methylethyl)-3-nitrobenzenemethanol [$R^1=(CH_3)_2CH$ in formula (XVI)]

33 g (170.8 mmol) of 2-methyl-1-(3-nitrophenyl)propanone was charged into a 500 ml eggplant type flask. 100 ml of ethanol was added and the mixture was cooled on an ice water bath. To this, 4.2 g (111 mmol) of $NaBH_4$ was slowly added and the mixture was reacted for 30 minutes under ice cooling and for 2 hours at room temperature. The resulting reaction liquid was concentrated by evaporator, and the residue was washed with water to quantitatively obtain α-(1-methylethyl)-3-nitrobenzenemethanol as a light brown oily product.

d) Synthesis of 1-[(1-butoxy-2-methyl)propyl]-3-nitrobenzene [$R^1=(CH_3)_2CH$, $R^2=CH_3CH_2CH_2CH_2$ in formula (XIX)]

30 ml of n-hexane was charged into a 300 ml reaction flask, followed by the addition of 4.4 g (110 mmol) of 60% oily NaH. The mixture was thoroughly shaken to remove n-hexane by decantation. 30 ml of n-hexane was added to repeat the above procedure to remove oily component of NaH. 100 ml of DMF was added under ice cooling. To this was added 19.5 g (99.9 mmol) of α-(1-methylethyl)-3-nitrobenzene methanol in portions to convert it into Na-oxide. 20.6 g (150.3 mmol) of 1-bromo-n-butane was added dropwise and reacted for 30 minutes under ice cooling and for 2 hours at room temperature. The resulting reaction mixture was poured into ice water and extracted with ethyl acetate to obtain 23 g of a crude product. This crude product was purified by column chromatography using a 5:1 mixture of n-hexane and ethyl acetate as a solvent to obtain 18.5 g (73.6 mmol) of light brown oily product of 1-[(1-butoxy-2-methyl)propyl]-3-nitrobenzene at an yield of 73.7%.

e) Synthesis of 3-[(1-butoxy-2-methyl)propyl]aniline [$R^1=(CH_3)_2CH$, $R^2=CH_3CH_2CH_2CH_2$ in formula (II)]

12.5 g (49.7 mmol) of 1-[(1-butoxy-2-methyl)propyl]-3-nitrobenzene, 60 ml of ethanol, 1.2 g of activated charcoal, 0.3 g of $FeCl_3 \cdot 6H_2O$, and 1 ml of hydrazine hydrate were charged into a 300 ml reaction flask. After refluxing for 15 minutes, 12 ml of hydrazine hydrate was added dropwise over 30 minutes, followed by refluxing for a further 3 hours. The reaction liquid was aspirated through a filter paper to remove activated charcoal, and the residue was concentrated by evaporator and dissolved into ethyl acetate, to obtain 10 g a crude product. This crude product was purified by column chromatography using a 10:1 mixture of n-hexane and ethyl acetate as a solvent to obtain 8.5 g (38.4 mmol) of light yellow oily product of 3-[(1-butoxy-2-methyl)propyl]aniline at an yield of 77.3%.

**Formulation Example** 1 <Dust>

| | parts by weight |
|---|---|
| Compound 1 | 3 |
| Clay | 40 |
| Talc | 57 |

The above ingredients were pulverized and mixed to obtain a dust.

**Preparation Example 2** <Wettable Powder>

| | parts by weight |
|---|---|
| Compound 2 | 50 |
| Lignin sulfonate | 5 |
| Alkyl sulfonate | 3 |
| Diatomaceous earth | 42 |

The above ingredients were pulverized and mixed to obtain wettable powder.

**Preparation Example 3** <Granules>

| | parts by weight |
|---|---|
| Compound 6 | 5 |
| Bentonite | 43 |
| Clay | 45 |
| Lignin sulfonate | 7 |

The above ingredients were mixed and kneaded with the addition of water. The mixture was extruded and then dried to obtain granules.

**Preparation Example 4** <Emulsion Concentrate>

| | parts by weight |
|---|---|
| Compound 9 | 20 |
| Polyoxyethylene alkylaryl ether | 10 |
| Polyoxyethylenesorbitan monolaurate | 3 |
| Xylene | 67 |

The above ingredients were mixed and dissolved to obtain an emulsion concentrate.

**Test Example 1**

<Test for controlling effect against cucumber gray mold (Botrytis cinerea on cucumber)>

A wettable powder similar to that prepared in Formulation Example 2, diluted with water to a prescribed concentration, was sprayed onto the cucumber plants of the first leaf stage (variety: *SAGAMI HAMPAKU*) growing in an unglazed pot (diameter: 10 cm), in an amount of 5 ml per pot. After drying the sprayed leaves in the air, a circular cutting (diameter: 4 mm) of agar containing Botrytis cinerea, which had been cultured in advance in a potato-sucrose agar medium at 20°C for 3 days, was attached directly to the center part of the leaves, and the pot was maintained at 20-22°C under high humidity conditions. Three days after the inoculation, lesion areas of the disease were examined to calculate the inhibition rate according to the following equation.

$$\text{Inhibition Rate (\%)} = \left[ 1 - \frac{\text{Lesion area in treated plot}}{\text{Lesion area in untreated plot}} \right] \times 100$$

The results are shown in Table 2.

**Test Example 2**

<Test for controlling effect against cucumber downy mildew *(Pseudoperonospora cubensis* on cucumber)>

A wettable powder similar to that prepared in Formulation Example 2, diluted with water to a prescribed concentration, was sprayed onto the cucumber plants of the second-leaf stage (variety: *SAGAMI HAMUPAKU*, one plant per pot and 3 pots were used in the treated plot) growing in an unglazed pot (diameter: 10 cm), in an amount of 5 ml per pot. After drying the sprayed leaves in the air, a suspension of the spores of *Pseudoperonospora cubensis,* which had been collected from contracted leaf of cucumber, was sprayed onto the leaves, and the pots were maintained at 20-22°C under high humidity conditions for 24 hours. 5-7 days after the inoculation, lesion areas of the disease were examined to calculate the inhibition rate according to the above equation.

The results are shown in Table 2.

**Test Example 3**

<Test for controlling effect against tomato late blight *(Phytophthora infestans* on tomato)>

A wettable powder similar to that prepared in Formulation Example 2, diluted with water to a prescribed concentration, was sprayed onto the young seedlings of tomato of the third leaf stage (variety: *FUKUJU* No. 2, one plant per pot and 3 pots were used in the treated plot) growing in an unglazed pot (diameter: 10 cm), in an amount of 5 ml per pot. After drying the sprayed leaves in the air, a suspension of the spores of *Phytophthora infestans,* which had been collected from contracted leaf of tomato, was sprayed onto the leaves, and the pots were maintained at 20-22°C under high humidity conditions for 24 hours, followed by growing in a greenhouse. 5-7 days after the inoculation, lesion areas of the disease were examined to calculate the inhibition rate according to the above equation.

The results are shown in Table 2.

**Test Example 4**

<Test for controlling effect against wheat red rust *(Puccinia recondita* on wheat)>

A wettable powder similar to that prepared in Formulation Example 2, diluted with water to a prescribed concentration, was sprayed onto the young seedlings of wheat of the second leaf stage (variety: *NORIN* No. 64, 16 seedlings per pot) growing in an unglazed pot (diameter: 10 cm), in an amount of 5 ml per pot. After drying the sprayed leaves in the air, a suspension of the uredospores of *Puccinia recondita,* which had been collected from contracted leaf of wheat, was sprayed onto the leaves, and the pots were maintained at 20-23°C under high humidity conditions for 24 hours, followed by growing in a greenhouse. 7-10 days after the inoculation, lesion areas of the disease were examined to calculate the inhibition rate according to the above equation.

The results are shown in Table 2.

**Test Example 5**

<Test for controlling effect against wheat powdery mildew *(Erysiphe graminis f. sp. tritici* on wheat)>

A wettable powder similar to that prepared in Formulation Example 2, diluted with water to a prescribed concentration, was sprayed onto the young seedlings of wheat of the second leaf stage (variety: *NORIN* No. 64, 16 seedlings per pot) growing in an unglazed pot (diameter: 10 cm), in an amount of 5 ml per pot. After drying the sprayed leaves in the air, a suspension of the underspores of *Exysiphe graminis f. sp. tritici*, which had been collected from the attacked leaf of wheat, was sprayed onto the leaves, and the pots were maintained at 20-24°C under high humidity conditions for 24 hours, followed by growing in a greenhouse. 9-11 days after the inoculation, lesion areas of the disease were examined to calculate the inhibition rate according to the above equation.

The results are shown in Table 2.

TABLE 2

| | Inhibition rate (%) at concentration of 500 ppm | | | | |
|---|---|---|---|---|---|
| Compound No. | Cucumber gray mold | Cucumber downy mildew | Tomato late blight | Wheat leaf rust | Wheat powdery mildew |
| 1 | 72 | | | | |
| 2 | 88 | | | | |
| 3 | 73 | 66 | 55 | 57 | 55 |
| 4 | 57 | 70 | | | |

TABLE 2 (continued)

| | Inhibition rate (%) at concentration of 500 ppm | | | | |
|---|---|---|---|---|---|
| Compound No. | Cucumber gray mold | Cucumber downy mildew | Tomato late blight | Wheat leaf rust | Wheat powdery mildew |
| 5 | 82 | | | | |
| 6 | 80 | | | | |
| 7 | 47 | | | | |
| 8 | 72 | | | | |
| 9 | 90 | | | | |
| 10 | 49 | | | | |
| 11 | 58 | | | | |

**Claims**

**1.** A derivative of 1-phenyl-5-substituted-1H-1,2,4-triazole-3-carboxamide represented by the following formula (I),

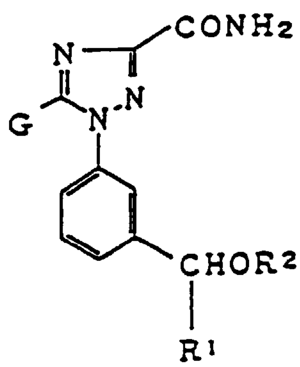

(I )

wherein $R^1$ is a $C_1$-$C_6$ alkyl group, $R^2$ is a $C_1$-$C_8$ alkyl group, and G represents a $C_1$-$C_8$ alkyl group, a $C_1$-$C_4$ fluoroalkyl group, a $C_3$-$C_7$ cycloalkyl group, a phenyl $C_1$-$C_4$ alkyl group, a phenyl $C_2$-$C_4$ alkenyl group, a morpholino group, or a thenyl group.

**2.** A derivative according to claim 1, **characterized** in that

$R^1 = (CH_3)_2CH$,
$R^2 = CH_3CH_2CH_2CH_2$ and
G = $(CH_3)_2CHCH_2$, cyclohexyl, $PhCH_2$ or $PhCH_2CH_2$.

**3.** A derivative according to claim 1 or 2, **characterized** in that it is 1-[3-[(1-Butoxy-2-methyl)propyl]phenyl]-1,2,4-triazole-3-carboxamide.

**4.** A process for manufacturing said derivative of 1-phenyl-5-substituted-1H-1,2,4-triazole-3-carboxamide, which comprises reacting oxazoledione hydrazone derivative (VI) and ammonia to produce glycineamide derivative (VII) and subjecting the glycineamide derivative (VII) to a cyclocondensation reaction according to the following reaction formula (I),

**Reaction (I)**

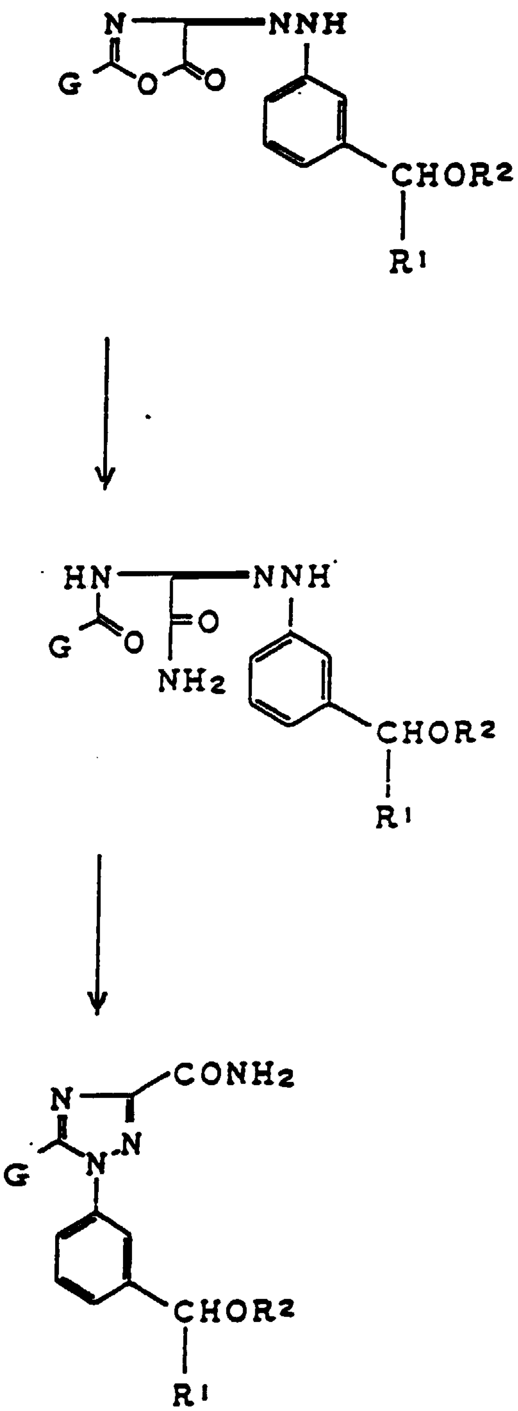

(VI)

(VII )

(I )

wherein $R^1$ is a $C_1$-$C_6$ alkyl group, $R^2$ is a $C_1$-$C_8$ alkyl group, and G represents a $C_1$-$C_8$ alkyl group, a $C_1$-$C_4$ fluoroalkyl group, a $C_3$-$C_7$ cycloalkyl group, a phenyl $C_1$-$C_4$ alkyl group, a phenyl $C_2$-$C_4$ alkenyl group, a morpholino group, or a thenyl group.

**5.** A process for manufacturing said derivative of 1-phenyl-5-substituted-1H-1,2,4-triazole-3-carboxamide, which comprises reacting oxamide derivative (X) and aldehyde derivative (XI) to produce dihydrotriazole carboxamide (XII) and oxidizing the dihydrotriazole carboxamide (XII) according to the following reaction formula (II),

Reaction (II)

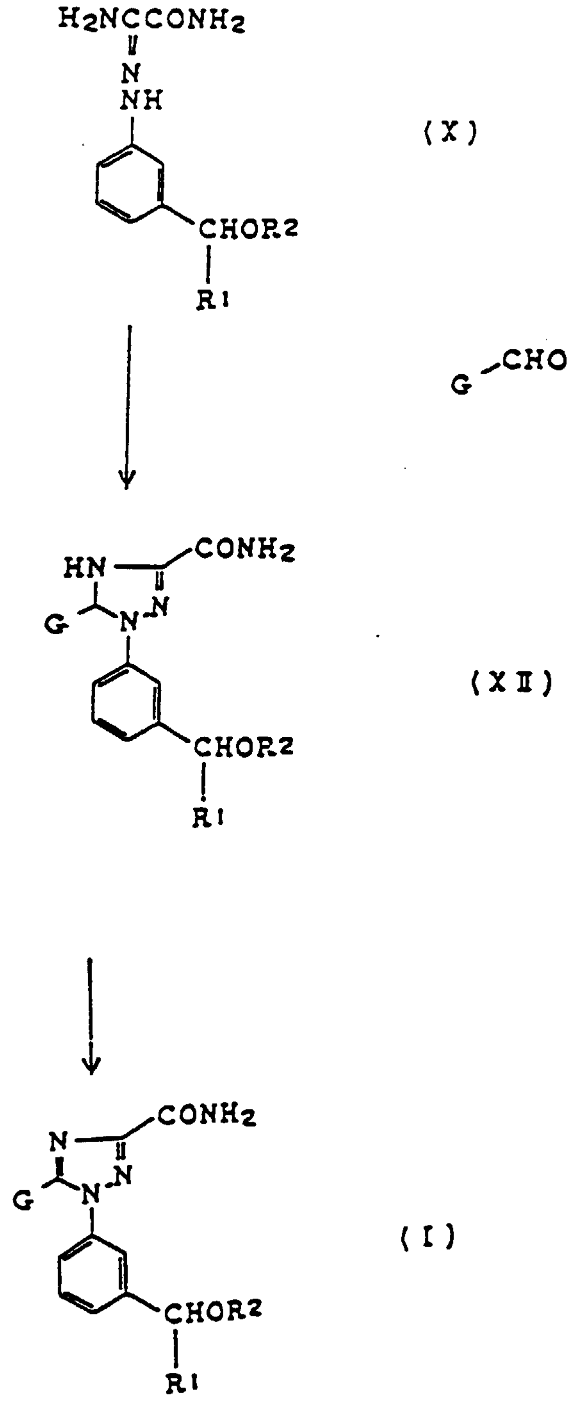

(X)

(XI)

(XII)

(I)

wherein $R^1$ is a $C_1$-$C_6$ alkyl group, $R^2$ is a $C_1$-$C_8$ alkyl group, and G represents a $C_1$-$C_8$ alkyl group, a $C_1$-$C_4$ fluoroalkyl group, a $C_3$-$C_7$ cycloalkyl group, a phenyl $C_1$-$C_4$ alkyl group, a phenyl $C_2$-$C_4$ alkenyl group, a morpholino group, or a thenyl group.

**6.** A fungicidal composition comprising the derivative of 1-phenyl-5-substituted-1H-1,2,4-triazole-3-carboxamide (I) defined in Claim 1 as an active ingredient.

**7.** A fungicidal composition for agriculture and horticulture, comprising the derivative of 1-phenyl-5-substituted-1H-1,2,4-triazole-3-carboxamide (I) defined in Claim 1 and a carrier.

**8.** A fungicidal composition according to Claim 7, which is effective for cucumber gray mold.

**Patentansprüche**

**1.** Derivat von 1-Phenyl-5-substituiertem-1H-1,2,4-triazol-3-carboxamid der folgenden Formel (I) :

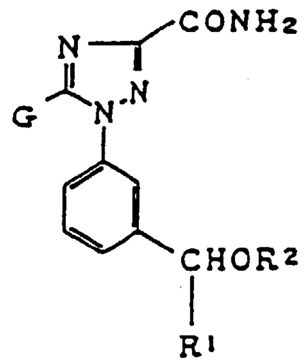

(I)

worin $R^1$ eine $C_{1-6}$-Alkylgruppe, $R^2$ eine $C_{1-8}$-Alkylgruppe und G eine $C_{1-8}$-Alkylgruppe, eine $C_{1-4}$-Fluoralkylgruppe, eine $C_{3-7}$-Cycloalkylgruppe, eine Phenyl-$C_{1-4}$-alkylgruppe, eine Phenyl-$C_{2-}$ 4-alkenylgruppe, eine Morpholinogruppe oder eine Thenylgruppe darstellt.

**2.** Derivat nach Anspruch 1, dadurch **gekennzeichnet**, daß

$R^1 = (CH_3)_2CH$,
$R^2 = CH_3CH_2CH_2CH_2$ und
$G = (CH_3)_2CHCH_2$, Cyclohexyl, $PhCH_2$ oder $PhCHCH_2$ ist.

**3.** Derivat nach Anspruch 1 oder 2, dadurch **gekennzeichnet**, daß es 1-[3-[(1-Butoxy-2-methyl)-propyl]-phenyl]-5-cyclohexyl-1H-1,2,4-triazol-3-carboxamid ist.

**4.** Verfahren zur Herstellung eines Derivats von 1-Phenyl-5-substituiertem-1H-1,2,4-triazol-3-carboxamid, bei dem man ein Oxazoldionhydrazon-Derivat (VI) und Ammoniak unter Bildung eines Glycinamid-Derivats (VII) umsetzt und das Glycinamid-Derivat (VII) einer Cyclokondensationsreaktion gemäß dem folgenden Reaktionsschema (I) unterwirft:

Reaktion (I)

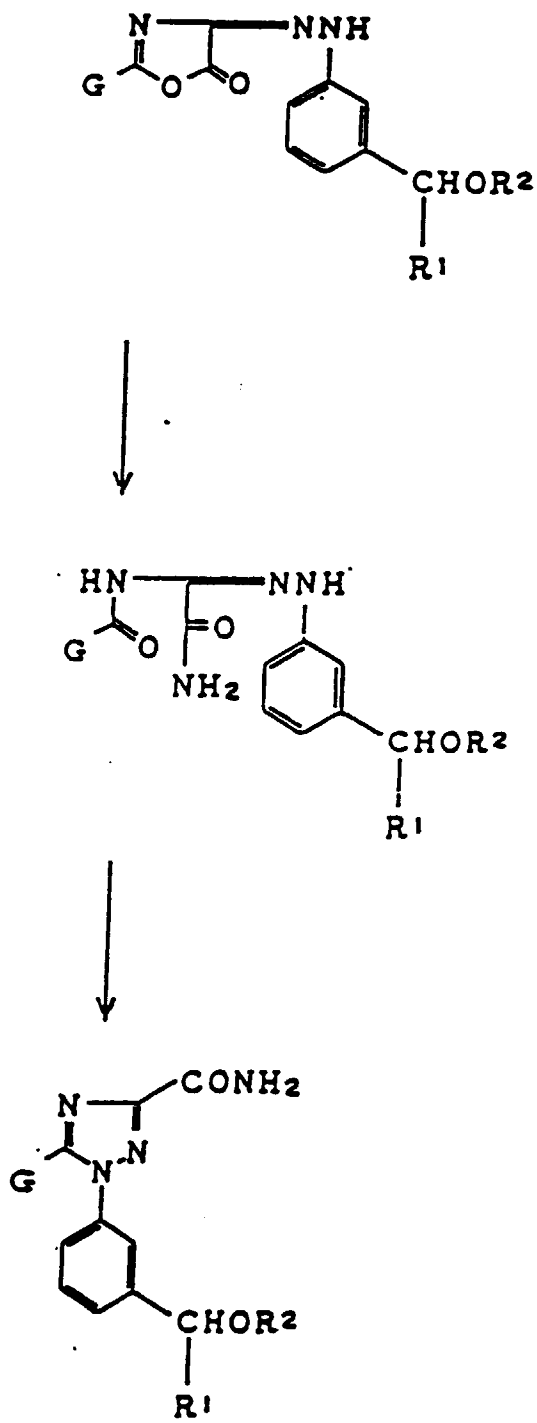

(VI)

(VII)

(I)

worin $R^1$ eine $C_{1-6}$-Alkylgruppe, $R^2$ eine $C_{1-8}$-Alkylgruppe und G eine $C_{1-8}$-Alkylgruppe, eine $C_{1-4}$-Fluoralkylgruppe,

eine $C_{3-7}$-Cycloalkylgruppe, eine Phenyl-$C_{1-4}$-alkylgruppe, eine Phenyl-$C_{2-4}$-alkenylgruppe, eine Morpholinogruppe oder eine Thenylgruppe darstellt.

5. Verfahren zur Herstellung eines Derivats von 1-Phenyl-5-substituiertem-1H-1,2,4-triazol-3-carboxamid, bei dem man ein Oxamid-Derivat (X) und ein Aldehyd-Derivat (XI) unter Bildung von Dihydrotriazolcarboxamid (XII) umsetzt und das Dihydrotriazolcarboxamid (XII) gemäß dem folgenden Reaktionsschema (II) oxidiert:

Reaktion II

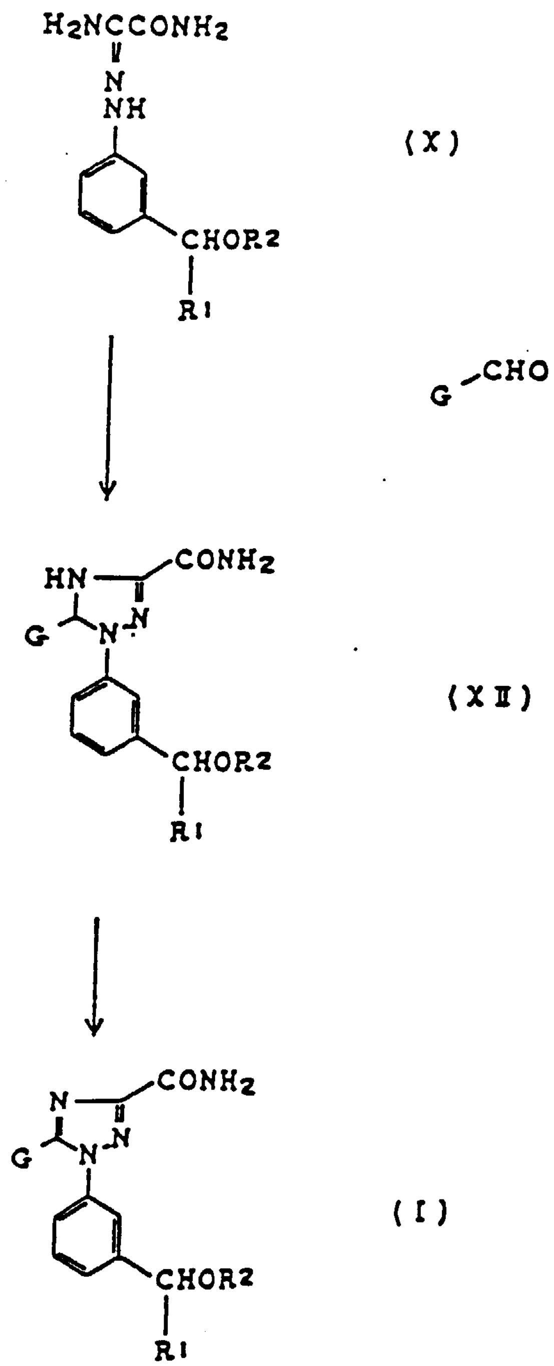

(X)

(XI)

(XII)

(I)

worin $R^1$ eine $C_{1-6}$-Alkylgruppe, $R^2$ eine $C_{1-8}$-Alkylgruppe und G eine $C_{1-8}$-Alkylgruppe, eine $C_{1-4}$-Fluoralkylgruppe, eine $C_{3-7}$-Cycloalkylgruppe, eine Phenyl-$C_{1-4}$-Alkylgruppe, eine Phenyl-$C_{2-4}$-Alkenylgruppe, eine Morpholinogruppe oder eine Thenylgruppe darstellt.

**6.** Fungicidale Zusammensetzung mit einem Derivat von 1-Phenyl-5-substituiertem-1H-1,2,4-triazol-3-carboxamid (I) gemäß Anspruch 1 als Wirkstoff.

**7.** Fungicidale Zusammensetzung für Gartenbau und Landwirtschaft mit einem Derivat von 1-Phenyl-5-substituiertem-1H-1,2,4-triazol-3-carboxamid (I) gemäß Anspruch 1 und einem Träger.

**8.** Fungicidale Zusammensetzung nach Anspruch 7 zur Bekämpfung vom grauem Gurkenschimmel (cucumber gray mold).

## Revendications

**1.** Dérivé du 1-phényl-1H-1,2,4-triazole-3-carboxamide substitué en 5 représenté par la formule suivante (I):

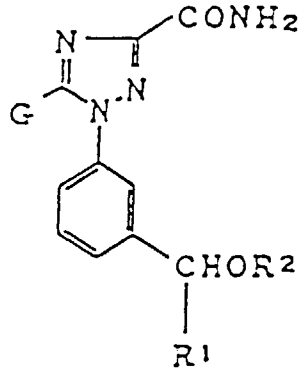

(I)

dans laquelle $R^1$ représente un groupe alkyle en $C_1$-$C_6$, $R^2$ représente un groupe alkyle en $C_1$-$C_8$, et G représente un groupe alkyle en $C_1$-$C_8$, un groupe fluoroalkyle en $C_1$-$C_4$, un groupe cycloalkyle en $C_3$-$C_7$, un groupe phényl (alkyle en $C_1$-$C_4$), un groupe phényl(alcényle en $C_2$-$C_4$), un groupe morpholino ou un groupe thényle.

**2.** Dérivé conforme à la revendication 1, caractérisé en ce que

$R^1 = (CH_3)_2CH$,
$R_2 = CH_3CH_2CH_2CH_2$ et
G = $(CH_3)_2CHCH_2$, cyclohexyl, $PhCH_2$ ou $PhCHCH_2$.

**3.** Dérivé conforme à la revendication 1 ou 2, caractérisé en ce qu'il est le 1-[3-[(1-butoxy-2-méthyl)propyl]phényl]-5-cyclohexyl-1H-1,2,4-triazole-3-carboxamide.

**4.** Procédé pour fabriquer ledit dérivé du 1-phényl-1H-1,2,4-triazole-3-carboxamide substitué en 5, qui comprend le fait de faire réagir un dérivé d'oxazoledione-hydrazone (VI) avec le l'ammoniac afin de produire un dérivé de glycine-amide (VII), et le fait de soumettre le dérivé de glycine-amide (VII) à une réaction de condensation cyclique, conformément au schéma réactionnel (I) suivant:

Réaction (I) :

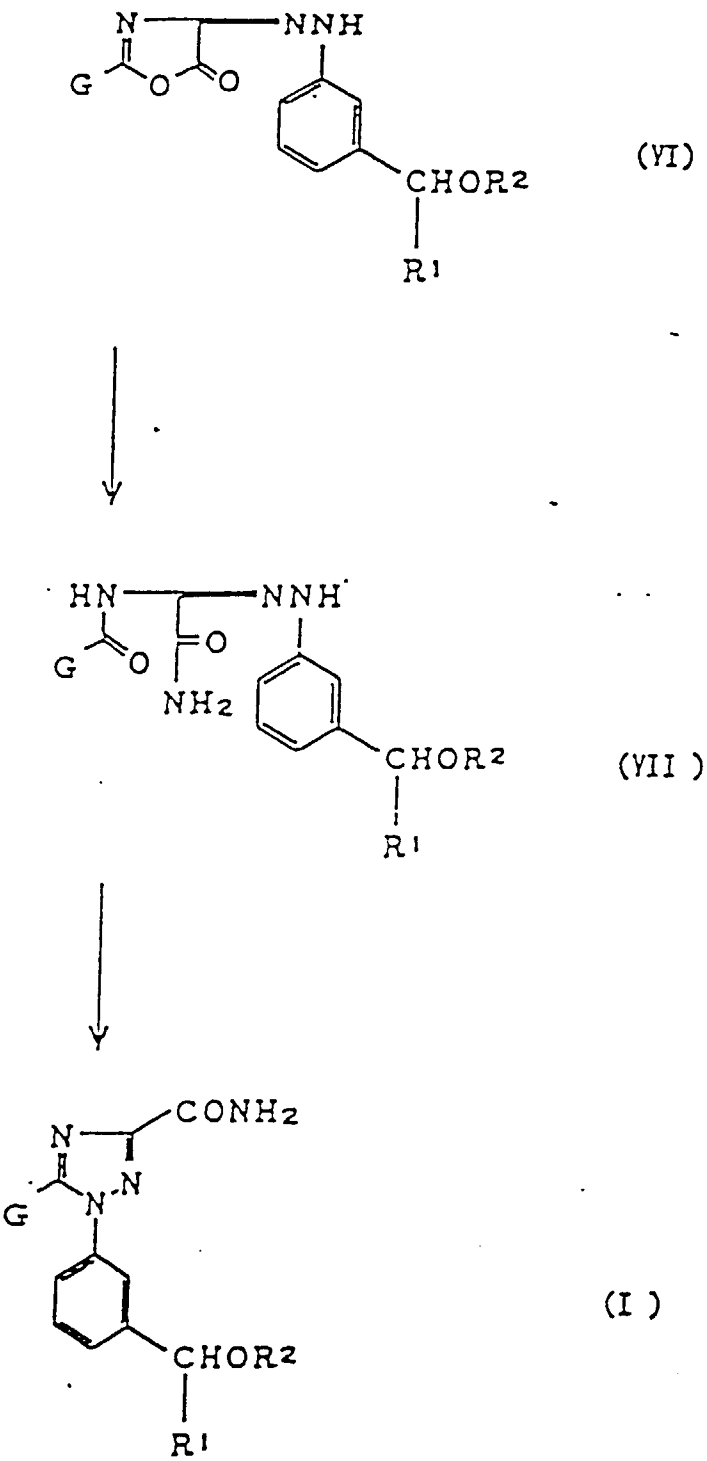

sachant que $R^1$ représente un groupe alkyle en $C_1$-$C_6$, $R^2$ représente un groupe alkyle en $C_1$-$C_8$, et G représente un groupe alkyle en $C_1$-$C_8$, un groupe fluoroalkyle en $C_1$-$C_4$, un groupe cycloalkyle en $C_3$-$C_7$, un groupe phényl (alkyle en $C_1$-$C_4$), un groupe phényl(alcényle en $C_2$-$C_4$), un groupe morpholino ou un groupe thényle.

**5.** Procédé pour fabriquer ledit dérivé du 1-phényl-1H-1,2,4-triazole-3-carboxamide substitué en 5, qui comprend le fait de faire réagir un dérivé d'oxamide (X) avec un dérivé d'aldéhyde (XI) afin de produire un dihydrotriazole-carboxamide (XII), et le fait d'oxyder le dihydrotriazole-carboxamide (XII), conformément au schéma réactionnel (II) suivant :

Réaction (II) :

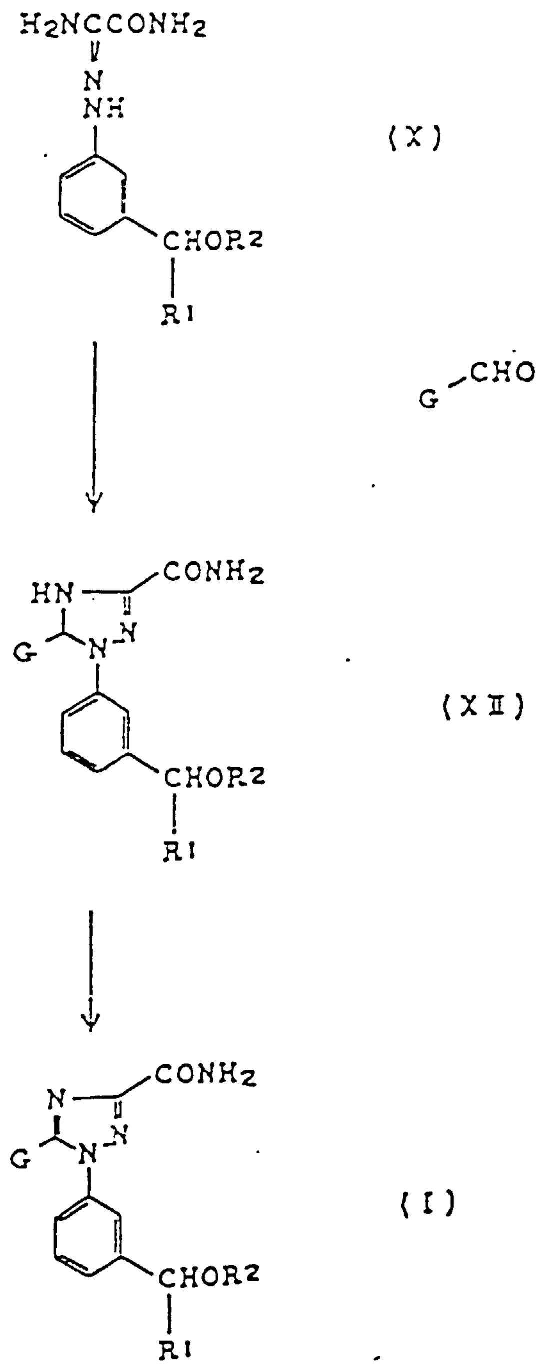

(X)

(XI)

(XII)

(I)

sachant que $R^1$ représente un groupe alkyle en $C_1$-$C_6$, $R^2$ représente un groupe alkyle en $C_1$-$C_8$, et G représente un groupe alkyle en $C_1$-$C_8$, un groupe fluoroalkyle en $C_1$-$C_4$, un groupe cycloalkyle en $C_3$-$C_7$, un groupe phényl (alkyle en $C_1$-$C_4$), un groupe phényl(alcényle en $C_2$-$C_4$), un groupe morpholino ou un groupe thényle.

6. Composition fongicide comprenant le dérivé du 1-phényl-1H-1,2,4-triazole-3-carboxamide substitué en 5 (I), défini dans la revendication 1, comme ingrédient actif.

7. Composition fongicide pour l'agriculture et l'horticulture, comprenant le dérivé du 1-phényl-1H-1,2,4-triazole-3-carboxamide substitué en 5 (I), défini dans la revendication 1, et un véhicule.

8. Composition fongicide conforme à la revendication 7, qui est efficace contre une pourriture grise de concombre.